# EUROPEAN PATENT SPECIFICATION

(11) **EP 3 237 518 B1**
(45) Date of publication and mention of the grant of the patent: **19.12.2018**
(21) Application number: 15817315.3
(22) Date of filing: 21.12.2015
(51) Int. Cl.: C08K 5/00, C09D 167/08

(54) **COATING COMPOSITION COMPRISING AN ALKYD-COMPRISING RESIN AND A DRIER**
BESCHICHTUNGSZUSAMMENSETZUNG MIT EINEM ALKYDHALTIGEN HARZ UND TROCKNER
COMPOSITION DE REVÊTEMENT COMPRENANT UNE RÉSINE AVEC DE L'ALKYDE ET UN SICCATIF

(30) Priority: 24.12.2014 EP 14200278
(43) Date of publication of application: 01.11.2017
(73) Proprietor: Akzo Nobel Coatings International B.V., 6824 BM Arnhem (NL)
(72) Inventor: MEIJER, Michel Daniel, NL-2324 MC Leiden (NL); RIJLAARSDAM, Eric Harry Jacobus, NL-2408 CG Alphen aan den Rijn (NL); VAN STREUN, Karel Hindrik, NL-6835 CT Arnhem (NL); HOGERVORST, Dave Matteus Adam Theodorus, NL-2182 VB Hillegom (NL)
(74) Representative: Akzo Nobel IP Department
(86) International application number: PCT/EP2015/080789
(87) International publication number: WO 2016/102464

(56) References cited:
- WO-A1-2014/095670
- US-A- 6 051 633
- OYMAN Z O ET AL: "A promising environmentally-friendly manganese-based catalyst for alkyd emulsion coatings", POLYMER, ELSEVIER SCIENCE PUBLISHERS B.V, GB, vol. 45, no. 22, 13 October 2004 (2004-10-13), pages 7431-7436, XP004595792, ISSN: 0032-3861, DOI: 10.1016/J.POLYMER.2004.08.052

## Description

### Field of the Invention

The present invention relates to a coating composition comprising an alkyd-comprising resin and a drier comprising a dinuclear ligand-manganese complex comprising manganese and a 1,4,7-trisubstituted-1,4,7-triazacyclononane ligand.

### Background of the Invention

Alkyd resins are widely used in coating compositions such as paint. An alkyd is a fatty acid functionalized polyester resin that comprises unsaturated fatty acids, such as for example oleic acid, linoleic acid, or linolenic acid. The fatty acid moieties of the alkyd resin react with oxygen from the atmosphere to form hydroperoxides which subsequently decompose to form free radicals. Reaction of these free radicals with the unsaturated carbon-carbon bonds of the fatty acid moieties causes covalent bonds to be formed between the alkyd polymer chains, thus forming cross-links between polymer chains. In this way, a liquid coating composition that comprises alkyd resin hardens to form a solid cured coating. This process is also referred to as auto-oxidation or drying.

The time for such a composition to dry depends on the concentration and type of unsaturated oil or fatty acids used to prepare the resin. Autoxidation and crosslinking of the unsaturated oil/fatty acid component can proceed unaided, but the time for drying is generally found to be unacceptably long for many practical purposes. The reactions are significantly accelerated by the presence of a metal-based drying catalyst, commonly referred to as a "drier". Whereas an alkyd coating may takes months to dry in the absence of a drying catalyst, in the presence of such a catalyst, drying can be accomplished within a few hours. The metal within the drying catalyst catalyzes autoxidation by forming a complex with both atmospheric oxygen and the double bonds of the unsaturated fatty acid groups within the composition.

Examples of known drier salts include polyvalent salts containing cobalt, calcium, copper, zinc, iron, zirconium, manganese, barium, zinc, strontium, lithium and potassium as the cation; and halides, nitrates, sulphates, carboxylates, such as acetates, ethylhexanoates, octanoates and naphthenates, or acetoacetonates as the anion. The catalytic activity of the metal during decomposition of the (hydro)peroxide relies on the repeated transition of the metal ion from the lower to the higher oxidation state and back again, leading to reduction and oxidation of the hydroperoxides to catalyze and accelerate oxidation of the unsaturated oil component of the composition. For this reason, transition metals are commonly employed in such driers, as transition metals are capable of switching from a lower valence state to a higher valence state in a redox reaction with fatty acid peroxides present in the alkyd composition.

To date, driers based on cobalt have been most widely used because of their good performance at ambient temperature. However, because of suspected carcinogenicity and/or toxicity of cobalt salts, it is now desired to find alternative drier compounds that show at least comparable drying performance to that of cobalt driers and which can replace cobalt based driers completely in air-drying coatings.

Driers based on non-cobalt metals, in particular driers comprising dinuclear complexes of manganese and a ligand, are known from *inter alia* WO2011/098584, WO2011/098583, WO2011/098587, or Oyman et al., Polymer 45 (2004), p. 7431-7436.

Manganese-based driers have, however, the drawbacks that they may not promote sufficient drying in a coating composition comprising an alkyd resin, especially in relation to tack free time, and that they can yield coatings which suffer from severe dark yellowing. In WO 2013/092441 is disclosed a coating composition comprising an alkyd resin and a dinuclear ligand-manganese complex as drier that is obtained by adding to the coating composition a manganese salt of the general formula Mn²⁺[X]ₙ, and a ligand that is a 1,4,7-trisubstituted-1,4,7-triazacyclononane. The ligand and manganese salt are added to the coating composition in such amounts that the molar ratio of ligand to manganese is at least 1.25. The coating composition of WO 2013/092441, i.e. with excess ligand, shows less yellowing than coating compositions to which ligand and manganese salt were added in a ligand to manganese ratio of 1.0 or lower. It is also shown in WO 2013/092441 that replacing part of the 1,4,7-trisubstituted-1,4,7-triazacyclononane by other amine ligands results in coating compositions that perform less well in terms of yellowing and drying. Coating composition comprising an alkyd resin and a dinuclear ligand-manganese complex comprising manganese and a 1,4,7-trisubstituted-1,4,7-triazacyclononane are also known from WO2014/095670.

Although the use of driers comprising a dinuclear complex of manganese and a 1,4,7-trisubstituted-1,4,7-triazacyclononane ligand already result in improved coating compositions in terms of yellowing and drying, in particular in case excess ligand is used, there is still room for improvement, in particular with regard to dark yellowing and drying properties. Therefore, there is a need in the art for coating compositions comprising an alkyd-comprising resin and a manganese drier that has improved properties.

### Summary of the Invention

It has now been found that if, in a coating composition comprising an alkyd-comprising resin and a drier comprising a dinuclear complex of manganese and a 1,4,7-trisubstituted-1,4,7-triazacyclononane ligand, the alkyd-comprising resin is an alkyd-stabilized non-aqueous dispersion of particles of addition polymer in a non-aqueous liquid phase comprising alkyd, the coating composition exhibits surprisingly less dark yellowing compared to compositions wherein other types of alkyd resins are used.

Accordingly, the present invention relates to a coating composition comprising an alkyd-comprising resin and a drier comprising a dinuclear ligand-manganese complex comprising manganese and a 1,4,7-trisubstituted-1,4,7-triazacyclononane ligand, wherein the alkyd-comprising resin is an alkyd-stabilized non-aqueous dispersion of particles of addition polymer in a non-aqueous liquid phase comprising alkyd.

It has, moreover, been found that the coating composition shows good drying properties.

### Detailed Description of the Invention

The coating composition according to the invention comprises an alkyd-comprising resin and a drier comprising a dinuclear complex of manganese and a 1,4,7-trisubstituted-1,4,7-triazacyclononane ligand. Preferably, the ligand is a 1,4,7-trisubstituted-1,4,7-triazacyclononane ligand having the general structure: 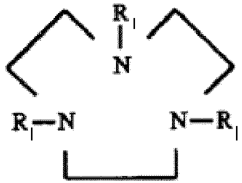 wherein R₁ is a C₁-C₂₀ alkyl, optionally substituted with heteroatoms, or a C₆-C₂₀ aryl, optionally substituted with heteroatoms. More preferably the ligand is a 1,4,7-trialkyl-1,4,7-triazacyclononane wherein R₁ is a C₁-C₂₀ alkyl, optionally substituted with heteroatoms. Even more preferably, the ligand is 1,4,7-trimethyl-1,4,7-triazacyclononane.

Dinuclear complex of manganese and a 1,4,7-substituted-1,4,7-triazacyclononane ligand are known and comprise two manganese nuclei, each bound to a 1,4,7-substituted-1,4,7-triazacyclononane ligand. The manganese nuclei each have, independently, a I, II, III, or IV valence state and are bound to each other by means of three bridges. Typically the bridges are, independently, an oxo or a carboxylate bridge.

Preferably, in the coating composition according to the invention, the drier comprises the dinuclear ligand-manganese complex and an additional amount of the ligand. More preferably, the drier comprises dinuclear ligand-manganese complex and additional ligand in such amounts that the molar ratio of ligand to manganese is at least 1.25, even more preferably at least 2.0, still more preferably at least 5.0. The molar ratio of ligand to manganese is preferably at most 30, more preferably at most 20.

The dinuclear ligand-manganese complex may be manufactured as such and then added to a coating composition to obtain the coating composition according to the invention. Preferably, the drier is a drier obtainable by:
- providing a manganese salt having the general formula Mn²⁺[X]ₙ, wherein X is an anion having a charge of n-, wherein n is 1 or 2;
- providing the ligand; and
- mixing the manganese salt with the ligand.

Preferably, the manganese salt and the ligand are mixed in such amounts that the molar ratio of ligand to manganese is in excess of 1, more preferably at least 1.25, even more preferably at least 2.0, still more preferably at least 5.0. The manganese salt and the ligand are preferably mixed in such amounts that the molar ratio of ligand to manganese is at most 30, more preferably at most 20. An advantage of an excess ligand is that the coating composition shows less yellowing.

X is preferably an anion selected from PF₆⁻, SbF₆⁻, AsF₆-, BF₄⁻, B(C₆F₅)⁻, Cl⁻, B⁻, I⁻, NO₃⁻, or R₂COO⁻ in which case n is 2, or X is SO₄²⁻ in which case n is 1. More preferably X is R₂COO⁻, n is 2, and R₂ is a C₁-C₂₀ alkyl, optionally substituted with heteroatoms. A particular preferred manganese salt is manganese octanoate, more in particular manganese ethylhexanoate.

If the drier is obtained by mixing such manganese salt with the ligand, such mixing may be carried out just before the mixture thus obtained is added to a coating composition to obtain the coating composition according to the invention. Preferably, such manganese salt and the ligand are separately added to the coating composition, under mixing, such that the mixing occurs in the coating composition.

The coating composition may comprise further driers, often referred to as secondary driers. Such driers are well known in the art. Such secondary driers are typically polyvalent salts containing: barium, zirconium, calcium, bismuth, copper, zinc, iron, potassium, strontium neodymium, sodium or lithium as the cation; and, halides, nitrates, sulphates, carboxylates like acetates, ethylhexanoates, octanoates and naphthenates or acetoacetonates as the anion. Metallic soaps, which are soluble in the binder of the coating composition, may in particular be mentioned in this regard; examples of such soaps, which may be used individually or in combination, include strontium octoate, copper octoate, zirconium octoate, zinc octoate and calcium octoate.

Preferably, the coating composition does not comprise a calcium-based drier.

The coating composition may comprise, in addition to the dinuclear ligand-manganese complex, further primary driers, preferably a cobalt-free primary drier. Preferably such further primary drier is a complex of iron and a tridentate, tetradentate, pentadentate or hexadentate nitrogen donor ligand, preferably a bispidine ligand. A particular preferred bispidine ligand is a bispidon, also known as di-substituted 3-methyl-9-oxo-2,4-di(pyridine-2-yl)-7-(pyridine-2-ylmethyl)-3,7-diazabicyclo[3.3.1]nonane-1,5-dicarboxylate. A particularly preferred ligand is dimethyl 3-methyl-9-oxo-2,4-di(pyridine-2-yl)-7-(pyridine-2-ylmethyl)-3,7-diazabicyclo[3.3.1]nonane-1,5-dicarboxylate.

The iron in the iron-ligand complex may have any suitable oxidation state. In complexes with a bispidine or bispidon ligand, iron is preferably in its 2+ oxidation state, i.e. Fe(II). The coating composition preferably comprises an iron(II) complex of di-substituted 3-methyl-9-oxo-2,4-di(pyridine-2-yl)-7-(pyridine-2-ylmethyl)-3,7-diazabicyclo[3.3.1]nonane-1,5-dicarboxylate as further primary drier, more preferably [(bispidon)Fe(II)Cl]Cl or [(bispidon)Fe(II)SO₄], even more preferably [(bispidon)Fe(II)Cl]Cl, which is also known as iron(1+), chloro[dimethyl-9,9-dihydroxy-3-methyl-2,4-di(2-pyridyl-κN)-7-[(2-pyridinyl-κN)methyl]-3,7-diazabicyclo[3.3.1]nonane-1,5-dicarboxylate-κN3, κN7]-, chloride(1-). This complex is registered under CAS nr. 478945-46-9 and is commercially available in solution as Borchi ® Oxy-Coat from OMG Borchers.

Preferably, the coating composition does not comprise a cobalt-based primary drier.

The coating composition may comprise the dinuclear complex of manganese and a 1,4,7-trisubstituted-1,4,7-triazacyclononane ligand and any further primary and/or secondary driers in any suitable amounts. The total amount of drier (primary and secondary) in the coating composition should typically not exceed 10 wt%, based on the total resin weight (alkyd and addition polymer), and preferably is in the range from 0.001-3 wt%, more preferably in the range of from 0.01 to 3 wt%, based on total resin weight. The amount of primary manganese drier is calculated as manganese plus ligand.

The alkyd-comprising resin is an alkyd-stabilized non-aqueous dispersion of particles of addition polymer in a non-aqueous liquid phase comprising alkyd.

Alkyd-stabilized non-aqueous dispersions (NADs) of particles of addition polymer are known in the art, for example from US 6,501,633, US 4,983,716, and US 5,516,820. Such NADs are typically prepared by first providing a liquid alkyd medium, and then adding to the alkyd medium one or more monomers that can form an addition polymer that is substantially insoluble in the alkyd medium, and allowing the monomers to form an addition polymer by a free radical polymerization reaction. The alkyd medium thus acts as polymerization medium and may for example be a pure alkyd, an alkyd dissolved in a one or more non-polar solvents, or an alkyd/oil mixture. The relatively polar addition polymer thus-formed will form particles in the relatively non-polar alkyd medium. Thus, the alkyd medium serves as dispersing medium for the particles of addition polymer and part of the alkyd from the medium will act as an alkyd stabilizer to stabilize the dispersed particles.

The coating composition according to the invention may comprise any suitable alkyd-stabilized non-aqueous dispersion of particles of addition polymer known in the art, for example an alkyd-stabilized non-aqueous dispersion of particles of addition polymer as disclosed in US 6,051,633.

Preferably the alkyd-stabilized non-aqueous dispersion of particles of addition polymer is obtainable by a process comprising the following steps:
a) providing a liquid alkyd medium;
b) adding to the alkyd medium one or more ethylenically unsaturated monomers; and
c) polymerizing the one or more monomers by means of free radical polymerization.

The liquid alkyd medium that is provided in step a) may be a substantially pure alkyd, a mixture of two or more substantially pure alkyds, one or more alkyds diluted by a solvent or a mixture of one or more alkyds and oil. Preferably, the alkyd medium comprises at least 25 wt% alkyd, more preferably at least 30 wt% alkyd, even more preferably at least 40 wt% alkyd. The alkyd medium may comprise up to 100 wt% alkyd.

If the alkyd medium comprises one of more alkyds diluted in a solvent, any suitable solvent may be used, for example a hydrocarbon, ketone, ester or alcohol solvent. Preferably the solvent is an aliphatic hydrocarbon solvent, a ketone solvent, or an ester solvent.

The one or more alkyds in the alkyd medium preferably have a number averaged molecular weight in the range of from 1,000 to 10,000, more preferably of from 1,500 to 5,000, even more preferably of from 2,000 to 4,000. Preferably the one or more alkyds have a polydispersity in the range of from 2 to 20, more preferably of from 2 to 10, even more preferably of from 2 to 6.

The one or more alkyds may be formed by any process known in the art using any raw material known in the art, for example by the reaction of a polyhydric alcohol, a polybasic acid and an unsaturated oil or fatty acid to give an unsaturated fatty acid residue containing ester.

Examples of suitable dihydric alcohols include ethylene glycol, 1,3-propane diol, 1,6-hexane diol, 1,12-dodecane diol, 3-methyl-1,5-pentane diol, 2,2,4-trimethyl-1,6-hexane diol, 2,2-dimethyl-1,3-propane diol, and 2-methyl-2-cyclohexyl-1,3-propane diol. Examples of suitable triols are glycerol, trimethylol ethane, and trimethylol propane. Suitable polyols having more than three hydroxyl groups are pentaerythritol, sorbitol, and etherification products of the compounds in question, such as ditrimethylol propane and di-, tri-, and tetrapentaerythritol.

Examples of suitable polybasic acids include phthalic acid, citric acid, fumaric acid, mesaconic acid, maleic acid, citraconic acid, isophthalic acid, terephthalic acid, 5-tert. butyl isophthalic acid, trimellitic acid, pyromellitic acid, succinic acid, adipic acid, 2,2,4-trimethyl adipic acid, azelaic acid, sebacic acid, dimerized fatty acids, cyclopentane-1,2-dicarboxylic acid, cyclohexane-1,2-dicarboxylic acid, 4-methylcyclohexane-1,2-dicarboxylic acid, tetrahydrophthalic acid, endomethylene-cyclohexane-1,2-dicarboxylic acid, butane-1,2,3,4-tetracarboxylic acid, endoisopropylidene-cyclohexane-1,2-dicarboxylic acid, cyclohexane-1,2,4,5-tetracarboxylic acid, and butane-1,2,3,4-tetracarboxylic acid. If so desired, the carboxylic acids in question may be used as anhydrides or in the form of an ester, preferably an ester of an alcohol having one to four carbon atoms.

Examples of suitable unsaturated fatty acids include ethylenically unsaturated conjugated or non-conjugated C₁₂-C₂₄ carboxylic acids, such as myristoleic, palmitoleic, arachidonic, erucic, gadoleic, clupanadonic, oleic, ricinoleic, linoleic, linolenic, licanic, nisinic acid and eleostearic acids or mixtures of two or more thereof, typically in the form of mixtures of fatty acids derived from natural or synthetic oils. Suitable unsaturated fatty acids for providing fatty acid groups in the alkyd also include fatty acids derived from soybean oil, conjugated soybean oil, palm oil, linseed oil, tung oil, rapeseed oil, sunflower oil, conjugated sunflower oil, calendula oil, wood oil, tallow oil, (dehydrated) castor oil, safflower oil, tuna fish oil, coconut oil and dehydrated coconut oil, and combinations thereof. Preferably fatty acids derived from sun flower oil or soya oil are used, such as oleic acid, linoleic acid, alpha-linolenic acid and mixtures of two or more thereof.

Preferably the one or more alkyds comprise at least 20 wt%, more preferably at least 50 wt%, even more at least 70 wt% of unsaturated fatty acid residues based on the total weight of the alkyd. Preferably, the one or more alkyds comprise at most 90 wt% unsaturated fatty acid residues, more preferably at most 85 wt%.

A specific example of a suitable alkyd is the condensation product of soya oil, phthalic anhydride, and pentaerythritol.

Optionally, the one or more alkyds may comprise other building blocks, which can for example be derived from monocarboxylic acids such as pivalic acid, 2-ethylhexanoic acid, lauric acid, palmitic acid, stearic acid, 4-tert. butyl-benzoic acid, cyclopentane carboxylic acid, naphthenic acid, cyclohexane carboxylic acid, 2,4-dimethyl benzoic acid, 2-methyl benzoic acid, benzoic acid, 2,2-dimethylol propionic acid, tetrahydrobenzoic acid, and hydrogenated or non-hydrogenated abietic acid or its isomer. If so desired, the monocarboxylic acids in question may be used wholly or in part as triglyceride, e.g. as vegetable oil, in the preparation of the alkyd. If so desired, mixtures of two or more of such monocarboxylic acids or triglycerides may be employed.

Optionally, isocyanates may also be used as building blocks for the alkyd. Suitable isocyanates include diisocyanates, such as 1,6-hexane diisocyanate, isophorone diisocyanate, toluene diisocyanate, diphenyl diisocyanate, and dicyclo-hexylmethane diisocyanate, and triisocyanates.

Preferably the alkyd has a final polymer acid value of from 1 to 20 mg KOH/g alkyd.

The one or more monomers that are added to the alkyd medium to form the addition polymer particles may be any monomers which are able to produce a polymer via a free radical addition mechanism. Such monomers are monomers comprising an ethylenically unsaturated group, more preferably a vinyl group, even more an acrylate group. Preferably, the one or more ethylenically unsaturated monomers comprise one or more acrylate monomers.

Examples of suitable monomers include acrylonitrile, methacrylonitrile, methyl methacrylate, methyl acrylate, ethyl acrylate, ethyl methacrylate, butyl acrylate, butyl methacrylate, hydroxyethyl acrylate, hydroxylethyl methacrylate, hydroxypropyl acrylate, hydroxylpropyl methacrylate, lauryl acrylate, lauryl methacrylate, trimethylol propane triacrylate, trimethylol propane trimethacrylate, hexanediol diacrylate, polyethylene oxide acrylate, polypropylene oxide acrylate, polypropylene oxide methacrylate allyl alcohol, acrylamide, methacrylamide, vinyl chloride, vinylidene chloride, and mixtures of two or more thereof.

Preferably at least one of the monomers comprises a polar functional group such as an acrylonitrile, acrylamide, alkyleneoxide or hydroxy functional group, more preferably a hydroxy functional group. Examples of suitable monomers comprising a hydroxy functional group are hydroxyethyl acrylate, hydroxyethyl methacrylate, hydroxypropyl acrylate, and hydroxypropyl methacrylate. More preferably, the one or more monomers are a mixture of monomers comprising a hydroxy functional group and monomers free of such hydroxy functional group, such as for example a mixture comprising hydroxyethyl acrylate and methyl methacrylate.

In case the one of more monomers added to form the addition polymer comprise monomers with a hydroxy functional group and monomers free of such hydroxy functional group, preferably in the range of from 5 to 35 wt% of the total weight of monomers added has a hydroxy functional group, more preferably of from 10 to 30 wt%.

In steps b) and c), the one or more monomers are added to the alkyd medium and the one or more monomers are polymerized by means of free radical polymerization. Conditions that allow the monomers added to the alkyd medium to polymerize into an addition polymer by free radical polymerization are well-known in the art. Any suitable conditions may be applied. Suitable conditions typically include the presence of an initiator, the presence a chain transfer agent and a temperature that is sufficient to allow polymerization. Preferably, the temperature of the alkyd medium during polymerization step c) is in the range of from 60 °C to 150 °C, more preferably of from 70 °C to 140 °C, even more preferably of from 75 °C to 120 °C.

Any suitable initiator may be used in a suitable amount. Suitable initiators are known in the art and include organic peroxides and nitrile initiators such as for example azobisisobutyronitrile. Suitable organic peroxides include benzoyl peroxide, lauroyl peroxide, di-t-butyl peroxide, acetyl peroxide, t-butyl peroctonate, t-amyl peroctonate, and t-butyl perbenzoate. The initiator may be added in any suitable amount, typically up to 3 wt% based on the total weight of the one or more monomers, preferably in the range of from 1.0 to 3.0 wt%, more preferably of from 1.5 to 2.5 wt%. Preferably the total amount of initiator is added in two or three steps, i.e. an amount at the start of the addition polymerization and a further amount during the polymerization reaction. More preferably, an organic peroxide is added as initiator at the start of the addition polymerization and a weaker initiator, such as a nitrile initiator is added during the polymerization reaction. The staged addition of initiator with the addition of a weaker initiator during the reaction, i.e. at a stage wherein less unreacted monomers are present, minimizes undesired polymerization reactions of the alkyd present in the polymerization medium.

Any suitable chain transfer agent may be used in a suitable amount. Suitable chain transfer agents are known in the art and include methyl mercaptoproprionate, dodecyl mercaptan, thioglycolic acid, 2-mercapto ethanol, and butenediol. Preferably, a chain transfer agent that does not comprise a mercapto group is used. A particularly preferred chain transfer agent is 2-butene-1,4-diol.

Optionally a polymerization catalyst is used during polymerization step c). Suitable polymerization catalysts are known in the art (often referred to as 'activators').

The one of more monomers are preferably gradually added to the alkyd medium during polymerization step c). More preferably the one or more monomers are dropwise added to the alkyd medium during polymerization step c). The monomers may be added as such, i.e. as pure monomers. Alternatively, the monomers may be dispersed in an amount of alkyd or solvent or a mixture thereof.

After polymerization step c), a dispersion of particles of addition polymer in the alkyd medium is formed. The alkyd medium thus serves as dispersing medium for the particles of addition polymer and part of the alkyd from the alkyd medium will act as an alkyd stabilizer to stabilize the dispersed particles.

The addition polymer particles may have any suitable size; preferably the particles have an average diameter in the range of from 100 to 800 nm, more preferably of from 200 to 700 nm, more preferably of from 300 to 600 nm.

The coating composition according to the invention comprises the alkyd-stabilized non-aqueous dispersion of particles of addition polymer in a liquid phase comprising alkyd and a drier comprising a dinuclear ligand-manganese complex.

The non-aqueous liquid phase comprising alkyd may be the alkyd medium wherein the addition polymerization took place as such. Preferably, additional alkyd is added to the alkyd medium after a non-aqueous dispersion of particles of addition polymer is formed in the alkyd medium in step c). Preferably, the coating composition comprises in the range of from 10 to 50 wt% alkyd, preferably of from 20 to 45 wt% alkyd, more preferably of from 25 to 40 wt% alkyd. The weight ratio of addition polymer to alkyd in the coating composition is preferably of from 0.1 to 10, more preferably of from 0.2 to 5.0, more preferably of from 0.3 to 2.0. The non-aqueous liquid phase may comprise a non-aqueous solvent. Such solvent may already be comprised in the alkyd medium provided in step a). Alternatively or additionally, solvent may be added to the alkyd medium after polymerization step c). Any suitable solvent may be used, preferably a solvent comprising aliphatic hydrocarbons, such as for example Shellsol D40, a ketone or an ester. If the coating composition comprises a solvent, the amount of solvent is preferably such that the amount of volatile organic compounds (VOC) in the coating composition does not exceed 400 grams per liter, more preferably does not exceed 300 grams per liter, even more preferably does not exceed 250 grams per liter.

The coating composition may comprise further ingredients that are known to be suitable ingredients for coating compositions, in particular for paint, including but not limited to crosslinking agents, catalysts, thickeners, extenders, color pigments, anti-skinning agents, dispersants, surfactants, biocides, and defoaming agents. Suitable further ingredients are known in the art and are for example disclosed in US 6,051,633.

The coating composition preferably is a paint composition. The paint composition may be a clear, colored or white paint composition. More preferably, the coating composition is a white paint composition.

### Examples

The invention is now further illustrated by means of the following non-limiting examples.

### Preparation of Alkyd No. 1

An alkyd was prepared by reacting a mixture of fatty acids derived from sunflower oil, phthalic anhydride (12 parts) and pentaerythritol (17 parts). The resulting alkyd (Alkyd No. 1) had an acid value lower than 10 mg KOH/g alkyd, an oil length of 74%, and a viscosity of 6 Pa.s (measured at 23 °C by a cone and plate method at 10,000 s⁻¹ at an alkyd content of 90% in Shellsol D40 as solvent).

### Preparation of Alkyd No.2

An alkyd was prepared in the same way as described for Alkyd No. 1, but now with oleic fatty acid instead of fatty acids derived from sunflower. The resulting alkyd (Alkyd No. 2) had an acid value lower than 10 mg KOH/g alkyd, an oil length of 74%, and a viscosity of 6 Pa.s (measured at 23 °C by a cone and plate method at 10,000 s⁻¹ at an alkyd content of 90% in Shellsol D40 as solvent).

### Preparation of Alkyd No. 3

An alkyd was prepared in the same way as described for Alkyd No. 1.

### Preparation of NAD No. 1

A non-aqueous dispersion of alkyd-stabilized acrylate polymer particles (NAD No. 1) was prepared as follows:
An acrylate monomer mixture was prepared by mixing methyl methacrylate (278 parts), 2-hydroxypropylacrylate (95 parts), 2-hydroxyethylmethacrylate (189 parts) and methacrylic acid (30 parts).

A solution of Alkyd No. 1 (148 parts) in Shellsol D40 solvent (166 parts) was added to a 4-necked reactor equipped with a stirrer, inert gas, and a reflux condenser. To the alkyd solution was added 2-butene-1,4-diol (12 parts) as initiator and part of the mixture of acrylate monomers (59 parts). This reactor was heated to 50°C under stirring under a nitrogen blanked. Then t-butyl peroxy-2-ethylhexanoate was dropwise added to the reaction mixture during 5 minutes (2 parts). The temperature of the reaction mixture was slowly heated to 100°C. Then using separate feeding lines, further solution of Alkyd No. 1 (102 parts) in D40 solvent (111 parts), and another part of the mixture of acrylate monomers (532 parts) were dropwise added during 3 hours. Simultaneously, a mixture of tert-butyl peroxy-2-ethylhexanoate (4 parts) and D40 solvent (40 parts) was slowly added to the reaction mixture during 3.25 hours. All feeding lines were flushing using D40 solvent (total 79 parts) and the reaction mixture was cooled to 95°C. Then, a solution of D40 solvent (51 parts), propylene glycol methyl ether acetate (51 parts) and 2,2'-azobis(2-methylbutyronitrile) (18 parts) was dropwise added during 1.5 hour. Hereafter, the mixture was allowed to react for another hour at 95°C, cooled to 50°C and filtered over a nylon filter bag (80µm) under a nitrogen blanket to obtain NAD No.1.

### Preparation of NAD No. 2

A non-aqueous dispersion of alkyd-stabilized acrylate polymer particles (NAD No. 2) was prepared as described for NAD No. 1 but now in a solution of Alkyd No. 2 instead of in a solution of Alkyd No. 1.

### Preparation of NAD No. 3

A non-aqueous dispersion of alkyd-stabilized acrylate polymer particles (NAD No. 3) was prepared as follows:
An acrylate monomer mixture was prepared by mixing methyl methacrylate (278 parts), 2-hydroxypropylacrylate (95 parts), 2-hydroxyethylmethacrylate (189 parts) and methacrylic acid (30 parts).

A solution of Alkyd No. 3 (193 parts) in Shellsol D40 solvent (300 parts) was added to a 4-necked reactor equipped with a stirrer, inert gas, and a reflux condenser. To the alkyd solution was added 2-butene-1,4-diol (12 parts) as initiator and part of the mixture of acrylate monomers (59 parts). This reactor was heated to 50°C under stirring under a nitrogen blanked. Then t-butyl peroxy-3,5,5-trimethylhexanoate was dropwise added to the reaction mixture during 5 minutes (2 parts). The temperature of the reaction mixture was slowly heated to 125°C. Then using separate feeding lines, further solution of Alkyd No. 1 (138 parts) in D40 solvent (200 parts), and another part of the mixture of acrylate monomers (532 parts) were dropwise added during 3 hours. Simultaneously, a mixture of t-butyl peroxy-3,5,5-trimethylhexanoate (5 parts) and D40 solvent (23 parts) was slowly added to the reaction mixture during 3 hours. The reaction mixture was cooled to 100°C. Then, a solution of D40 solvent (10 parts), propylene glycol methyl ether acetate (50 parts) and 2,2'-azobis(2-methylbutyronitrile) (6 parts) was dropwise added during 1.5 hour. Hereafter, the mixture was allowed to react for another hour at 100°C. Then, the reaction mixture was heated to 130°C and solvent was distilled in vacuum to a content of non-volatile compounds of 65%. The mixture was cooled to 50°C and filtered over a nylon filter bag (80µm) under a nitrogen blanket to obtain NAD No.3.

The properties of the NAD No. 1, NAD No. 2 and NAD No 3. are given in Table 1.

**Table 1 Properties of NADs**

| | NAD No. 1 | NAD No. 2 | NAD No. 3 |
|---|---|---|---|
| Content of non-volatile compounds (%) | 60.6 | 59.7 | 65.1 |
| Particle size (nm) | 361 | 403 | 373 |
| Cone & plate viscosity at 100 Pa, at 23°C (Pa.s) | 0.20 | 0.07 | 0.57 |
| Acid number of total polymer (mg KOH/g) | 24 | 23 | 24 |
| OH-number of total polymer (mg KOH/g) | 169 | 163 | 157 |

### Example 1 (comparative)

A white paint was prepared as follows:
A titanium dioxide slurry was prepared by adding TiO₂ powder (Kronos 2310, 21.3 parts) to a stirred mixture of Alkyd No. 1 (5.3 parts), Bentone clay (0.3 parts) and a carboxylic acid dispersant polymer (1 part), and dispersing using a high speed dissolver until a fineness below 10µm was obtained. To this slurry were added, under stirring, 60 parts of Alkyd No. 1, 4 parts of methylethylketoxime (MEKO), and a drier.

As drier were added: a solution of manganese ethylhexanoate and a solution of 1,4,7-trimethyl-1,4,7-triazacyclononane, Nuodex® Calcium 5% (calcium carboxylate), and Nuodex® Zirconium 18% (zirconium carboxylate) in such amounts that the paint composition comprised per 100 gram of paint composition 0.8 mg manganese, 38 mg ligand (1,4,7-trimethyl-1,4,7-triazacyclononane), 70 mg calcium, and 520 mg zirconium. The molar ratio of ligand to manganese was 18:1.

### Example 2 (invention)

A white paint was prepared as follows:
A titanium dioxide slurry was prepared by adding TiO₂ powder (Kronos 2310, 21.3 parts) to a stirred mixture of Alkyd No. 1 (5.3 parts), Bentone clay (0.3 parts) and a carboxylic acid dispersant polymer (1 part), and dispersing using a high speed dissolver until a fineness below 10µm was obtained. To this slurry was added, under stirring, Alkyd No. 1 (17.3 parts), NAD No. 1 (49.8 parts), 0.4 wt% MEKO (methyl ethyl ketone oxime) and a drier.

As primary drier were added to the resulting paint composition: a solution of manganese ethylhexanoate and a solution of 1,4,7-trimethyl-1,4,7-triazacyclononane. As secondary driers were added Nuodex® Calcium 5%, and Nuodex® Zirconium 18%. The driers were added in such amounts that the paint composition comprised per 100 gram of paint composition 0.8 mg manganese, 38 mg ligand (1,4,7-trimethyl-1,4,7-triazacyclononane), 70 mg calcium, and 520 mg zirconium. The molar ratio of ligand to manganese was 18:1.

### Example 3 (invention)

A white paint was prepared as in Example 2, except that no Nuodex® Calcium 5% was added as secondary drier. The driers were added in such amounts that the paint composition comprised per 100 gram of paint composition 0.8 mg manganese, 38 mg ligand (1,4,7-trimethyl-1,4,7-triazacyclononane), and 520 mg zirconium. The molar ratio of ligand to manganese was 18:1.

### Example 4 (invention)

A white paint was prepared as in Example 2, using NAD No. 2 instead of NAD No. 1.

### Example 5 (invention)

A white paint was prepared as in Example 3, using NAD No. 2 instead of NAD No. 1.

### Example 6 (invention)

A white paint was prepared as in Example 2, using NAD No. 3 instead of NAD No. 1.

### Example 7 (invention)

A white paint was prepared as in Example 3, using NAD No. 3 instead of NAD No. 1.

### Example 8 (invention)

A white paint was prepared as in Example 6, except that also Borchi ® Oxy-Coat (a 1% solution of iron(1+), chloro[dimethyl-9,9-dihydroxy-3-methyl-2,4-di(2-pyridyl-κN)-7-[(2-pyridinyl-κN)methyl]-3,7-diazabicyclo[3.3.1]nonane-1,5-dicarboxylate-κN3, κN7]-, chloride(1-)) was added as primary drier. The driers were added in such amounts that the paint composition comprised per 100 gram of paint composition 0.8 mg manganese, 38 mg ligand (1,4,7-trimethyl-1,4,7-triazacyclononane), 0.4 mg iron, 70 mg calcium, and 520 mg zirconium. The molar ratio of ligand to manganese was 18:1.

### Example 9 (invention)

A white paint was prepared as in Example 8, except that no Nuodex® Calcium 5% was added as secondary drier. The driers were added in such amounts that the paint composition comprised per 100 gram of paint composition 0.8 mg manganese, 38 mg ligand (1,4,7-trimethyl-1,4,7-triazacyclononane), 0.4 mg iron, and 520 mg zirconium. The molar ratio of ligand to manganese was 18:1.

### Paint properties

Dark yellowing and drying times of the white paints prepared in Examples 1 to 9 were determined as follows.

### Dark yellowing

Color characteristics of the white paints prepared in Examples 1 to 9 were measured on a Leneta opacity chart. The paint was applied on the chart using a rake with a radius of 0.7 mm and dried at 23°C overnight. Then, the color of the coatings was measured using a standard colorimeter. Then, the coatings were stored in the dark at 50°C for 500h and the color of the coatings (aged coatings) was measured again. The dark yellowing is defined as the difference in b*value according to CIE-lab color space between the freshly (overnight) dried coatings and the aged coatings. The whiteness index (WI) was calculated by using the b* and L*CIE-lab values measured on the aged coatings following the equation WI=L-3b.

### Drying time until surface dry at 10 °C

Drying times were determined by BK drying on a BK or drying recorder (wet film thickness 90 µm; ASTM D5895-96). After the application of the film on a glass strip (B.K. recorder: 69 x 2.5 cm) a vertical blunt needle, pressed upon by a 5 grams load, is placed into the freshly applied film and then dragged through the drying paint in a direction parallel to the edges of the strip.

The three stages of BK drying in the experiment were as follows: i) the (wet) paint flows together (leveling); b) the paint has begun to polymerize but a line left by the needle is visible or traceable (basis trace); and c) drying has proceeded sufficiently that the film of paint is not displaced by the needle (the so-called "surface dry time"). In Table 2 the drying time of the paint at 10 °C to achieve step c), i.e. the surface dry time, is given (in hours).

**Table 2 Paint properties**

| Example | Alkyd resin | Primary drier | Secondary drier | Dark yellowing 500 h | WI 500 h | Drying until surface dry (hours) |
|---|---|---|---|---|---|---|
| 1 (comp) | Alkyd No. 1 (no NAD) | Mn/TMTACN^{a} | Ca, Zr | 8.46 | 71.31 | 4.50 |
| 2 | NAD No. 1 | Mn/TMTACN | Ca, Zr | 6.05 | 78.87 | 3.50 |
| 3 | NAD No. 1 | Mn/TMTACN | Zr | 4.32 | 84.51 | 3.50 |
| 4 | NAD No. 2 | Mn/TMTACN | Ca, Zr | 5.06 | 82.00 | 4.50 |
| 5 | NAD No. 2 | Mn/TMTACN | Zr | 3.02 | 88.47 | 3.50 |
| 6 | NAD No. 3 | Mn/TMTACN | Ca, Zr | 5.82 | 71.60 | 5.8 |
| 7 | NAD No. 3 | Mn/TMTACN | Zr | 4.15 | 79.28 | 6.8 |
| 8 | NAD No. 3 | Mn/TMTACN, BOC^{b} | Ca, Zr | 5.45 | 71.08 | 3.0 |
| 9 | NAD No. 3 | Mn/TMTACN, BOC | Zr | 4.25 | 77.79 | 5.0 |

| | | | | | | |
|---|---|---|---|---|---|---|
| ^{a} TMTACN: 1,4,7-trimethyl-1,4,7-triazacyclononane ^{b} BOC: Borchi ® Oxy-Coat, ex. OMG Borchers | | | | | | |

## Claims

1. A coating composition comprising an alkyd-comprising resin and a drier comprising a dinuclear ligand-manganese complex comprising manganese and a 1,4,7-trisubstituted-1,4,7-triazacyclononane ligand, wherein the alkyd-comprising resin is an alkyd-stabilized non-aqueous dispersion of particles of addition polymer in a non-aqueous liquid phase comprising alkyd.

2. A coating composition according to claim 1, wherein the 1,4,7-trisubstituted-1,4,7-triazacyclononane ligand has the general structure 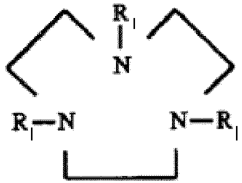 in which R₁ is a C₁-C₂₀ alkyl, optionally substituted with heteroatoms, or a C₆-C₂₀ aryl, optionally substituted with heteroatoms.

3. A coating composition according to claim 2, wherein the ligand is 1,4,7-trimethyl-1,4,7-triazacyclononane.

4. A coating composition according to any one of the preceding claims, wherein the drier comprises the dinuclear ligand-manganese complex and an additional amount of the ligand.

5. A coating composition according to any one of the preceding claims, wherein the drier is obtainable by:
- providing a manganese salt having the general formula Mn²⁺[X]ₙ, wherein X is an anion having a charge of n-, wherein n is 1 or 2;
- providing the ligand; and
- mixing the manganese salt with the ligand.

6. A coating composition according to claim 5, wherein X is R₂COO⁻, n is 2, and R₂ is a C₁-C₂₀ alkyl, optionally substituted with heteroatoms.

7. A coating composition according to claim 5 or 6, wherein the ligand is provided in an amount such that the molar ratio of ligand to manganese is at least 1.25:1, preferably at least 2.0:1.

8. A coating composition according to any one of the preceding claims, wherein the coating composition is free of a calcium-based drier.

9. A coating composition according to any one of the preceding claims, wherein the coating composition is free of a cobalt-based drier.

10. A coating composition according to any one of the preceding claims, wherein the drier further comprises a Fe(II) complex of disubstituted 3-methyl-9-oxo-2,4-di(pyridine-2-yl)-7-(pyridine-2-ylmethyl)-3,7-diazabicyclo[3.3.1]nonane-1,5-dicarboxylate.

11. A coating composition according to any one of the preceding claims, wherein the alkyd-stabilized non-aqueous dispersion of particles of addition polymer is obtainable by a process comprising the following steps:
a) providing a liquid alkyd medium;
b) adding to the alkyd medium one or more ethylenically unsaturated monomers; and
c) polymerizing the one or more monomers by means of free radical polymerization.

12. A coating composition according to claim 11, wherein the one or more ethylenically unsaturated monomers comprise one or more acrylate monomers.

13. A coating composition according to claim 12, wherein at least one of the one or more acrylate monomers is an acrylate monomer with a hydroxy functional group.

14. A coating composition according to claim 13, wherein the acrylate monomer with a hydroxy functional group is selected from the group consisting of hydroxyethyl acrylate, hydroxyethyl methacrylate, hydroxypropyl acrylate, and hydroxypropyl methacrylate.

15. A coating composition according to claim 13 or 14, wherein the one or more monomers further comprise a monomer that has no hydroxy functional groups.

## Patentansprüche

1. Beschichtungszusammensetzung, umfassend ein alkydhaltiges Harz und einen Trockner, umfassend einen dinuklearen Liganden-Mangan-Komplex, umfassend Mangan und einen 1,4,7-trisubstituierten-1,4,7-Triazacyclononan-Liganden, wobei das alkydhaltige Harz eine alkydstabilisierte, nicht wässrige Dispersion von Partikeln eines Additionspolymers in einer nicht wässrigen, flüssigen Phase, umfassend Alkyd, ist.

2. Beschichtungszusammensetzung nach Anspruch 1, wobei der 1,4,7-trisubstituierte-1,4,7-Triazacyclononan-Ligand die folgende allgemeine Struktur hat 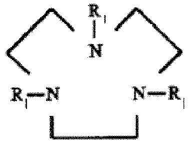 wobei R₁ ein C₁-C₂₀ Alkyl, wahlweise substituiert mit Heteroatomen, oder ein C₆-C₂₀ Aryl, wahlweise substituiert mit Heteroatomen, ist.

3. Beschichtungszusammensetzung nach Anspruch 2, wobei der Ligand 1,4,7-Trimethyl-1,4,7-Triazacyclononan ist.

4. Beschichtungszusammensetzung nach einem der vorhergehenden Ansprüche, wobei der Trockner den dinuklearen Liganden-Mangan-Komplex und eine zusätzliche Menge des Liganden umfasst.

5. Beschichtungszusammensetzung nach einem der vorhergehenden Ansprüche, wobei der Trockner erhältlich ist durch:
- Bereitstellen eines Mangansalzes mit der allgemeinen Formel Mn²⁺[X]ₙ, wobei X ein Anion mit einer Ladung von n- ist, wobei n gleich 1 oder 2 ist;
- Bereitstellen des Liganden; und
- Mischen des Mangansalzes mit dem Liganden.

6. Beschichtungszusammensetzung nach Anspruch 5, wobei X gleich R₂COO⁻, n gleich 2 und R₂ ein C₁-C₂₀ Alkyl, wahlweise substituiert mit Heteroatomen, ist.

7. Beschichtungszusammensetzung nach Anspruch 5 oder 6, wobei der Ligand in einer Menge bereitgestellt ist, so dass das Molverhältnis des Liganden zu dem Mangan wenigstens 1,25:1, vorzugsweise wenigstens 2,0:1 beträgt.

8. Beschichtungszusammensetzung nach einem der vorhergehenden Ansprüche, wobei die Beschichtungszusammensetzung frei von einem Trockner auf Kalziumbasis ist.

9. Beschichtungszusammensetzung nach einem der vorhergehenden Ansprüche, wobei die Beschichtungszusammensetzung frei von einem Trockner auf Kobaltbasis ist.

10. Beschichtungszusammensetzung nach einem der vorhergehenden Ansprüche, wobei der Trockner weiterhin einen Fe(II)-Komplex von disubstituiertem 3-Methyl-9-Oxo-2,4-Di(Pyridin-2-yl)-7-(Pyridin-2-ylmethyl)-3,7-Diazabicyclo[3.3.1]nonan-1,5-Dicarboxylat umfasst.

11. Beschichtungszusammensetzung nach einem der vorhergehenden Ansprüche, wobei die alkydstabilisierte, nicht wässrige Dispersion von Partikeln eines Additionspolymers durch ein Verfahren erhältlich ist, das die folgenden Schritte umfasst:
a) Bereitstellen eines flüssigen Alkyd-Mediums;
b) Hinzufügen von einem oder mehreren ethylenisch ungesättigten Monomeren zu dem Alkydmedium; und
c) Polymerisieren des einen oder der mehreren Monomere durch freie radikalische Polymerisation.

12. Beschichtungszusammensetzung nach Anspruch 11, wobei das eine oder die mehreren ethylenisch ungesättigten Monomere ein oder mehrere Acrylatmonomere umfassen.

13. Beschichtungszusammensetzung nach Anspruch 12, wobei wenigstens eines des einen oder der mehreren Acrylatmonomere ein Acrylatmonomer mit einer hydroxyfunktionellen Gruppe ist.

14. Beschichtungszusammensetzung nach Anspruch 13, wobei das Acrylatmonomer mit einer hydroxyfunktionellen Gruppe ausgewählt ist aus der Gruppe, bestehend aus Hydroxyethylacrylat, Hydroxyethylmethacrylat, Hydroxypropylacrylat und Hydroxypropylmethacrylat.

15. Beschichtungszusammensetzung nach Anspruch 13 oder 14, wobei das eine oder die mehreren Monomere weiterhin ein Monomer umfassen, das keine hydroxyfunktionellen Gruppen hat.

## Revendications

1. Composition de revêtement comprenant une résine comprenant un alkyde et un siccatif comprenant un complexe binucléaire ligand-manganèse comprenant du manganèse et un ligand 1,4,7-triazacyclononane trisubstitué en 1,4,7 où la résine comprenant un alkyde est une dispersion non aqueuse stabilisée par un alkyde de particules de polymère d'addition dans une phase liquide non aqueuse comprenant un alkyde.

2. Composition de revêtement selon la revendication 1, dans laquelle le ligand 1,4,7-triazacyclononane trisubstitué en 1,4,7 a la structure générale 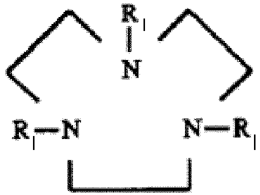 dans laquelle R₁ représente un alkyle en C₁ à C₂₀, éventuellement substitué par des hétéroatomes, ou un aryle en C₆ à C₂₀, éventuellement substitué par des hétéroatomes.

3. Composition de revêtement selon la revendication 2, dans laquelle le ligand est le 1,4,7-triméthyl-1,4,7-triazacyclononane.

4. Composition de revêtement selon l'une quelconque des revendications précédentes, dans laquelle le siccatif comprend le complexe binucléaire ligand-manganèse et une quantité supplémentaire du ligand.

5. Composition de revêtement selon l'une quelconque des revendications précédentes, dans laquelle le siccatif peut être obtenu :
- en fournissant un sel de manganèse ayant la formule générale Mn²⁺[X]ₙ, où X représente un anion ayant une charge de n-, où n est égal à 1 ou 2;
- en fournissant le ligand ; et
- en mélangeant le sel de manganèse avec le ligand.

6. Composition de revêtement selon la revendication 5, dans laquelle X représente R₂COO⁻, n est égal à 2 et R₂ représente un alkyle en C₁ à C₂₀, éventuellement substitué par des hétéroatomes.

7. Composition de revêtement selon la revendication 5 ou 6, dans laquelle le ligand est fourni en une quantité telle que le rapport molaire du ligand sur le manganèse est d'au moins 1,25:1, de préférence d'au moins 2,0:1.

8. Composition de revêtement selon l'une quelconque des revendications précédentes, dans laquelle la composition de revêtement est exempte de siccatif à base de calcium.

9. Composition de revêtement selon l'une quelconque des revendications précédentes, dans laquelle la composition de revêtement est exempte de siccatif à base de cobalt.

10. Composition de revêtement selon l'une quelconque des revendications précédentes, dans laquelle le siccatif comprend en outre un complexe de Fe(II) de 3-méthyl-9-oxo-2,4-di(pyridine-2-yl)-7-(pyridine-2-ylméthyl)-3,7-diazabicyclo[3.3.1]nonane-1,5-dicarboxylate disubstitué.

11. Composition de revêtement selon l'une quelconque des revendications précédentes, dans laquelle la dispersion non aqueuse stabilisée par un alkyde de particules de polymère d'addition peut être obtenue par un procédé comprenant les étapes suivantes qui consistent :
a) à fournir un milieu alkyde liquide ;
b) à ajouter au milieu alkyde un ou plusieurs monomère(s) à insaturation éthylénique ; et
c) à polymériser le ou les plusieurs monomère(s) par polymérisation radicalaire.

12. Composition de revêtement selon la revendication 11, dans laquelle le ou les plusieurs monomère(s) à insaturation éthylénique comprend/comprennent un ou plusieurs monomère(s) d'acrylate.

13. Composition de revêtement selon la revendication 12, dans laquelle au moins l'un du ou des plusieurs monomère(s) d'acrylate est un monomère d'acrylate ayant un groupe fonctionnel hydroxy.

14. Composition de revêtement selon la revendication 13, dans laquelle le monomère d'acrylate ayant un groupe fonctionnel hydroxy est choisi dans le groupe constitué par un acrylate d'hydroxyéthyle, un méthacrylate d'hydroxyéthyle, un acrylate d'hydroxypropyle et un méthacrylate d'hydroxypropyle.

15. Composition de revêtement selon la revendication 13 ou 14, dans laquelle le ou les plusieurs monomère(s) comprend/comprennent en outre un monomère qui n'a pas de groupe fonctionnel hydroxy.
